(19) Europäisches Patentamt
European Patent Office
Office européen des brevets

(11) Veröffentlichungsnummer: **0 072 531**
**A1**

# (12) EUROPÄISCHE PATENTANMELDUNG

(21) Anmeldenummer: 82107270.9

(22) Anmeldetag: 11.08.82

(51) Int. Cl.³: C 07 F 9/65
A 61 K 31/675

(30) Priorität: 14.08.81 DE 3132221

(43) Veröffentlichungstag der Anmeldung: 23.02.83 Patentblatt 83/8

(84) Benannte Vertragsstaaten: AT BE CH DE FR GB IT LI LU NL SE

(71) Anmelder: Max-Planck-Gesellschaft zur Förderung der Wissenschaften e.V.
Bunsenstrasse 10
D-3400 Göttingen(DE)

(71) Anmelder: BEHRINGWERKE AKTIENGESELLSCHAFT
Postfach 1140
D-3550 Marburg/Lahn(DE)

(72) Erfinder: Eibl, Hansjörg, Prof. Dr.
Steinweg 51
D-3406 Bovenden(DE)

(72) Erfinder: Kolar, Cenek, Dr.
Lindenweg 14
D-3550 Marburg/Lahn(DE)

(72) Erfinder: Seiler, Friedrich Robert
Oberer Eichweg 10
D-3550 Marburg/Lahn(DE)

(72) Erfinder: Sedlacek, Hans-Harald, Dr.
Am Sonnenhang 3
D-3550 Marburg/Lahn(DE)

(74) Vertreter: Meyer-Dulheuer, Karl-Hermann, Dr. et al,
HOECHST Aktiengesellschaft Zentrale Patentabteilung
Postfach 80 03 20
D-6230 Frankfurt/Main 80(DE)

## (54) Neue Cyclophosphamid-Derivate, Verfahren zu ihrer Herstellung und ihre Verwendung.

(57) Die Erfindung betrifft neue Cyclophosphamid-Derivate der Formel I

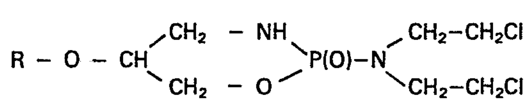

worin R ein Wasserstoffatom, einen Kohlenwasserstoffrest oder einen heterocyclischen Rest bedeutet. Die neuen Verbindungen zeigen eine dem Cyclophosphamid vergleichbare zytostatische Wirksamkeit bei einer wesentlich geringeren Toxizität.

EP 0 072 531 A1

MAX-PLANCK-GESELLSCHAFT ZUR FÖRDERUNG DER WISSENSCHAFTEN e.V.
und BEHRINGWERKE AKTIENGESELLSCHAFT HOE 81/B 750 Dr.HA

# Neue Cyclophosphamid-Derivate, Verfahren zu ihrer Herstellung und ihre Verwendung

Die Erfindung betrifft neue Cyclophosphamid-Derivate, ihre Herstellung und ihre Verwendung, insbesondere als Wirkstoff in Arzneimitteln.

Das Cyclophosphamid ist ein bewährtes Zytostatikum, welches unter verschiedenen Bezeichnungen im Handel erhältlich ist. Die cancerotoxische Selektivität der N-2-Chloräthylamido-oxaza-phosphorine, wie Cyclophosphamid (H. Arnold und F. Bourseaux, Angew. Chemie 7o (1958), 539) ist eng an die spezielle Ringstruktur des Oxaza-phosphorin-Ringes mit einer Propanolamin-Phosphorsäureester-Gruppierung gebunden.

Allgemein wird die Ansicht vertreten, daß sich die Lost-Amine im biologischen Milieu wie alkylierende Agenzien verhalten, indem sie mit aktiven Wasserstoffatomen, beispielsweise solchen von SH-Gruppen der am Zellaufbau beteiligten Proteine oder Enzyme, reagieren und so eine Wachstumshemmung oder Vernichtung der malignen Zelle verursachen. Der Verwendung der Lost-Amine in der Therapie sind jedoch enge Grenzen gesetzt, da sie auch normale Zellen angreifen und infolgedessen schon im Bereich wirksamer Dosen eine erhebliche Toxizität aufweisen. Der vorteilhaften zytostatischen Wirksamkeit steht somit eine erhebliche Toxizität gegenüber.

Der Erfindung liegt die Aufgabe zugrunde, Derivate des Cyclophosphamids zu schaffen, welche die erwünschte pharmakologische Wirksamkeit des Cyclophosphamids praktisch unverändert aufweisen, deren Toxizität gegenüber dem Cyclophosphamid jedoch wesentlich verringert ist, so daß die pharmakologische Breite verbessert wird.

Gelöst wird diese Aufgabe erfindungsgemäß durch neue Cyclophosphamid-Derivate der allgemeinen Formel I

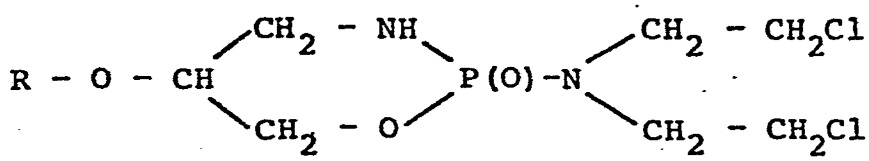

(I)

in der R ein Wasserstoffatom, einen Kohlenwasserstoffrest oder einen heterocyclischen Rest bedeutet.

Was wurde überraschenderweise gefunden, daß die Einführung eines über ein Sauerstoffatom gebundenen Kohlenwasserstoff- oder Heterocyclusrestes oder einer Hydroxylgruppe in Position 5 des Cyclophosphamidmoleküls die Toxizität wesentlich herabsetzt, ohne die zytostatische Wirksamkeit zu verringern.

Ein Kohlenwasserstoffrest ist vorzugsweise ein Alkylrest, ein Arylrest oder ein Aralkylrest. Ein Alkylrest kann geradkettig oder verzweigt sein und besitzt vorzugsweise 1 bis 26, insbesondere 1 bis 16 C-Atome, und kann eine oder mehrere Doppel- oder Dreifachbindungen oder/und eine oder mehrere OH-Gruppen enthalten. Der Alkylrest kann durch ein oder mehrere Sauerstoffatome unterbrochen sein oder/und auch aromatische oder nichtaromatische Ringsysteme, als Kettenglieder oder/und Substituenten enthalten, wie z. B. Phenyl oder Phenylen. Wenn der Alkylrest durch Sauerstoffatome unterbrochen ist, enthält er zwischen den Sauerstoffatomen vorzugsweise 2 bis 4 C-Atome. Ein Arylrest ist z. B. Naphthyl, und insbesondere Phenyl. Die Alkylengruppe in einem Aralkylrest leitet sich von einer wie oben definierten Alkylgruppe ab; sie besitzt insbesondere 1 bis 12 C-Atome. Ein Aralkylrest ist z. B. Phenäthyl oder Benzyl. Ein heterocyclischer Rest kann ein nichtaromatischer Rest, der sich von einem

alicyclischen Rest durch Ersatz eines oder mehrerer Glieder durch Heteroatome, wie z. B. -O-, -S-, -N=, ableitet, oder ein aromatischer heterocyclischer Rest sein.

Von den erfindungsgemäßen Verbindungen der allgemeinen Formel I sind besonders bevorzugt das 5-Hydroxy-cyclophosphamid (R = H) und die 5-Hydroxy-alkoxy-cyclophosphamide, insbesondere mit 1 bis 6 C-Atomen, und in denen die Hydroxygruppe vorzugsweise in der ω-Stellung steht. Abgesehen von ihrer Verwendung als Wirksubstanzen in Arzneimitteln sind die erfindungsgemäßen Verbindungen, vor allem die bevorzugt genannten, auch wertvolle Zwischenprodukte zur Herstellung weiterer Verbindungen mit insbesondere pharmakologischer Wirksamkeit.

Die erfindungsgemäßen neuen Verbindungen zeichnen sich dadurch aus, daß sie etwa die gleiche zytostatische Wirksamkeit aufweisen wie das Cyclophosphamid selbst, jedoch eine wesentlich verringerte Toxizität besitzen. So wurde beispielsweise für das 5-Hydroxy-phosphamid gefunden, daß die $ED_{50}$ der des Cyclophosphamids entspricht, die $LD_{50}$ aber etwa 10-mal so hoch ist wie beim Cyclophosphamid. Dies bedeutet eine Verbesserung des therapeutischen Index auf das 10-fache.

Gegenstand der Erfindung ist weiterhin ein Verfahren zur Herstellung der Verbindungen der Formel I, das dadurch gekennzeichnet ist, daß man eine Verbindung der allgemeinen Formel II

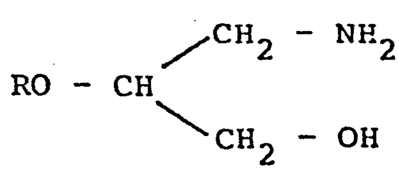

(II)

in der R die oben angegebene Bedeutung besitzt, in einem

organischen Lösungsmittel in Gegenwart einer Base mit N,N-Bis(2-chloräthyl)-phospho-amid-dichlorid oder mit Phosphoroxytrichlorid und Bis(2-chloräthyl)-amin-hydrochlorid umsetzt und, wenn erwünscht, die erhaltene Verbindung der allgemeinen Formel I nach an sich bekannten Verfahren in eine Verbindung überführt, in der R einen der anderen oben angegebenen Reste darstellt.

Die Umsetzung läßt sich unter Normalbedingungen in organischen Lösungsmitteln, wie chlorierten Kohlenwasserstoffen, Äthern, Tetrahydrofuran (THF), Dioxan, Dimethylformamid (DMF) oder Aromaten durchführen. Da bei der Umsetzung Chlorwasserstoff abgespalten wird, arbeitet man in Gegenwart einer Base, wie eines Amins. Bevorzugt werden Trialkylamine, wie Triäthylamin oder Tripropylamin und aromatische Stickstoffbasen, wie Pyridin.

Zur Herstellung der Verbindungen der Formel I, in der R ein Wasserstoffatom oder auch eine andere der angegebenen Bedeutungen besitzt, kann es zweckmäßig sein, von einer Verbindung der Formel II auszugehen, in der R eine Allyl- oder Benzylgruppe bedeutet. Im erhaltenen Cyclophosphamid-Derivat der Formel I kann die 5-Benzylgruppe durch katalytische Hydrogenolyse, die 5-Allylgruppe durch Umlagerung in das Propenyl-Derivat und Abspaltung in leicht saurem Medium entfernt werden.

Das erfindungsgemäße Verfahren führt beim Ringschluß stets zu zwei Verbindungen, die nach analytischen Kriterien identisch sind, sich aber durch eine unterschiedliche Konformation am Kohlenstoffatom 5, welches auch ein optisches Asymmetriezentrum darstellt, unterscheiden. Die beiden Konformationsisomeren ("endo"- und "exo"-Isomeren) lassen sich in einem geeigneten Lösungsmittelgemisch chromatographisch voneinander trennen.

Die Verbindungen der allgemeinen Formel II leiten sich vom Aminopropandiol ab und können z. B. nach einem der folgenden Verfahren hergestellt werden.

1. Ausgehend von optisch aktiven oder racemischen 1,2-O-Alkyliden-glycerin-Derivaten,

2. ausgehend von 2-O-Benzyl-glycerin,

3. ausgehend von Epichlorhydrin.

1. a) Eine Verbindung der Formel VI

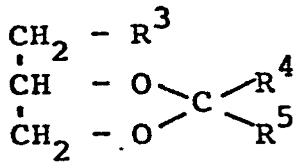

(VI)

in der $R^3$ eine Hydroxygruppe, $R^4$ und $R^5$ Wasserstoff, Alkyl oder Aryl bedeuten, wird mit einer acylierenden Verbindung, wie Methansulfonsäurechlorid oder p-Toluolsulfonsäurechlorid in an sich bekannter Weise zu dem Reaktionsprodukt der Formel VI, in der $R^3$ eine Acyloxygruppe, wie Methansulfonyloxy oder p-Toluolsulfonyloxy und $R^4$ und $R^5$ unverändert sind,

b) das Produkt von Stufe a) in an sich bekannter Weise mit Natriumazid in einem polaren Lösungsmittel, wie DMF oder Acetonitril, zu einem 1-Azido-glycerin-Derivat der Formel VI, worin $R^3$ eine Azidogruppe und $R^4$ und $R^5$ unverändert sind,

c) das Produkt von Stufe b) in an sich bekannter Weise zuerst mit einer Säure zu einer Verbindung der Formel VII

$$\begin{array}{l} CH_2 - N_3 \\ CH - OR^6 \\ CH_2 - OR^7 \end{array} \qquad (VII)$$

worin $R^6$ und $R^7$ Wasserstoffatome bedeuten, diese Verbindung selektiv zu einer Triphenylmethyläther-Verbindung der Formel VII, in der $R^6$ ein Wasserstoffatom und $R^7$ eine Triphenylmethylgruppe ist, und diese Verbindung zu einem 2-O-Arylalkyl- oder -Alkyl-, bevorzugt Benzyl-Derivat der Formel VII umgesetzt, worin $R^6$ eine Alkyl-, Arylalkyl- oder bevorzugt eine Benzylgruppe und $R^7$ eine Triphenylmethylgruppe bedeuten, und in dem erhaltenen Produkt die Triphenylmethylgruppe abgespalten, wodurch eine Verbindung der Formel VII entsteht, worin $R^6$ unverändert und $R^7$ ein Wasserstoffatom bedeuten, und

d) das Produkt der Stufe c) in an sich bekannter Weise in Gegenwart eines Hydrierungskatalysators hydriert oder mit einem Hydrid, wie Natrium-borhydrid oder Lithium-aluminiumhydrid, zu einer Verbindung der Formel II oder deren Hydrochlorid, worin R (= $R^6$) eine Alkyl- oder Arylalkyl-, bevorzugt eine Benzylgruppe, bedeutet, umgesetzt.

2. a) Eine Verbindung der Formel X

$$\begin{array}{l} CH_2 - R^9 \\ CH - OR^{10} \\ CH_2 - OR^{11} \end{array} \qquad (X)$$

in der $R^9$ eine Hydroxygruppe, $R^{10}$ eine Benzylgruppe und $R^{11}$ ein Wasserstoffatom sind, wird mit einer acylierenden Verbindung, wie Methan- oder p-Toluolsulfonylchlorid, in an sich bekannter Weise selektiv zu dem Reaktionsprodukt der Formel X, in der $R^9$ eine Acyloxygruppe und $R^{10}$ und $R^{11}$ unverändert sind,

b) das Produkt der Stufe a) in an sich bekannter Weise

mit Natriumazid in einem polaren Lösungsmittel, wie DMF oder Acetonitril, zu einem 1-Azido-Derivat der Formel X, worin $R^9$ eine Azidogruppe und $R^{10}$ und $R^{11}$ unverändert sind, und

c) das Produkt der Stufe b) in gleicher Weise wie in Stufe 1. d) zu einer Verbindung der Formel II umgesetzt.

3. a) Das Epichlorhydrin wird in an sich bekannter Weise mit Benzylalkohol in Gegenwart einer Base und das entstandene Produkt mit Natriumazid in Gegenwart von Ammoniumchlorid und einem polaren Lösungsmittel zu einer Verbindung der Formel XI

$$\begin{array}{l} CH_2 - N_3 \\ CH - OR^{12} \\ CH_2 - O\text{-Benzyl} \end{array} \qquad (XI)$$

worin $R^{12}$ ein Wasserstoffatom bedeutet,

b) das Produkt der Stufe a) in an sich bekannter Weise mit Methan- oder p-Toluolsulfonsäure-alkylester in Gegenwart einer Base, wie Kalium-tert.-butylat, und einem polaren Lösungsmittel, wie Dioxan oder DMF zu einer 2-O-Alkyl-Verbindung der Formel XI, worin $R^{12}$ eine Alkylgruppe ist, umgesetzt und

c) das Produkt der Stufe b) in an sich bekannter Weise in Gegenwart einer Säure, bevorzugt Salzsäure, unter Bildung einer Verbindung der Formel II bzw. deren Hydrochlorid, hydriert.

Die Verbindungen der Formel II, in denen R Benzyl oder Allyl bedeutet, lassen sich auch, wie in den Beispielen

beschrieben, ausgehend von 1-0-Allyl-2-0-benzyl-rac-glycerin, 1-0-Allyl-2-0-benzyl-sn-glycerin ($[\alpha]_D^{20} = -4,5^\circ$) oder 3-0-Allyl-2-0-benzyl-sn-glycerin ($[\alpha]_D^{20} = +4,5^\circ$) erhalten.

Gegenstand der Erfindung sind auch Arzneimittel, die eine oder mehrere der Verbindungen der Formel I als Wirkstoff enthalten. Neben den üblichen pharmazeutischen Konfektionierungs- und/oder Verdünnungsmitteln können diese Arzneimittel neben den Verbindungen der Formel I zur Unterstützung der Therapie gegebenenfalls auch noch weitere Wirkstoffe enthalten, sofern diese mit den erfindungsgemäßen Verbindungen der Formel I zusammen keine unerwünschten Nebenwirkungen zeigen. Die Dosierungs- und Anwendungsweise entspricht im wesentlichen den für das Cyclophosphamid bekannten Bedingungen, wobei aber aufgrund der besseren therapeutischen Breite (wesentlich geringere Toxizität) der erfindungsgemäßen Verbindungen auch höhere Dosierungen und/oder eine häufigere Verabreichung in Frage kommen.

Es kann auch zweckmäßig sein, eines der Konformationsisomeren ("endo"- oder "exo"-Form), und insbesondere einen der optischen Antipoden der Verbindungen der Formel I (Asymmetriezentrum in 5-Stellung) in angereicherter oder reiner Form einzusetzen. Die Trennung in die optischen Antipoden kann nach den dafür bekannten Methoden erfolgen.

Die folgenden Beispiele erläutern die Erfindung näher, ohne sie darauf zu beschränken.

Beispiel 1

3-Azido-3-desoxy-1,2-0-isopropyliden-sn-glycerin (1):

20 g (0.151 Mol) 1,2-0-Isopropyliden-sn-glycerin (H. Eibl, Chem. Phys. Lip. 1980, im Druck) werden in 150 ml trock. Pyridin gelöst. Unter Feuchtigkeitsausschluß werden bei 0°C 19.90 g (0.166 Mol) Methansulfonsäurechlorid zugetropft. Nach 12 h Rühren wird die Suspension i. vac. eingeengt, mit Toluol destilliert und mit 250 ml $CHCl_3$ versetzt. Nach viermaligem Waschen mit Eiswasser und verd. Salzsäure wird die org. Phase über Natriumsulfat getrocknet und eingeengt. Das dünnschichtchromatographisch reine Produkt ($CHCl_3$/Aceton 4:1, Ausbeute 30.8 g) wird in 120 ml trock. DMF gelöst und mit 9.75 g (0.15 Mol) Natriumazid 1.5 h bei 120°C erhitzt. Nach Abkühlen wird der Suspension 100 ml Wasser zugegeben und es wird mit Petrolether/Diisopropylether 1:1 extrahiert, mit Eiswasser nachgewaschen, die organische Phase mit Magnesiumsulfat getrocknet und zum Sirup eingeengt.

Ausbeute 23 g (92%) Kp. 123°C (0.05 Torr)

$[\alpha]_D^{20}$ = −72,7° (c = 0.5 in $CHCl_3$)

IR ($-N_3$; 2100 $cm^{-1}$)

$^{13}$C-NMR (270 MHz, $CDCl_3$):

C-1 $\delta$= 66.5, C-2 74.6, C-3 52.8 ppm

| | | | | |
|---|---|---|---|---|
| $C_6H_{11}N_3O_2$ (157.1) | Ber. | C 45.8 | H 7.05 | N 26.73 |
| | Gef. | C 45.6 | H 7.03 | N 26.64 |

Beispiel 2

3-Azido-3-desoxy-1-O-triphenylmethyl-sn-glycerin (2):

15.4 g (0.092 Mol) 1 werden mit 150 ml 60%iger Essigsäure 30 min. bei 80°-90°C erwärmt. Nach Einengen i. vac. wird der Sirup zweimal mit Toluol/Methanol 10:1 destilliert und Hochvak. getrocknet. Der erhaltene Sirup (12.2 g) wird in 100 ml trock. Pyridin gelöst und bei Zimmertemperatur mit 29.06 g (0.104 Mol) Chlortriphenylmethan versetzt. Nach 12 h wird i. vac. eingeengt, der Sirup in Chloroform aufgenommen und dreimal mit Wasser gewaschen. Das nach dem Einengen resultierende, dünnschichtchromatographisch (DC $CHCl_3$/Aceton 4:1) einheitliche Produkt wird noch an 200 g Kieselgel ($CHCl_3$/Aceton 8:1) gereinigt.
Ausbeute 29.3 g (90%)

$[\alpha]_D^{20} = -10.3°$ (c = 1 in $CHCl_3$)

IR ( $-N_3$; 2100 $cm^{-1}$, Ph 1500 $cm^{-1}$ und 1600 $cm^{-1}$)

$^{13}$C-NMR (270 MHz $CDCl_3$)

C-1 $\delta$ = 64.8   C-2 70.0   C-3 53.8 ppm

| | | | | |
|---|---|---|---|---|
| $C_{22}H_{21}N_3O_2$ (359.4) | Ber. | C 73.5 | H 5.89 | N 11.69 |
| | Gef. | C 73.5 | H 6.0 | N 11.45 |

Beispiel 3

3-Azido-2-O-benzyl-3-desoxy-sn-glycerin (3):

Zu 41.0 g (0.105 Mol) 2 gelöst in 200 ml trock. Dioxan werden 14.6 g Kaliumtertiärbutylat und 18.0 g (0.105 Mol) Benzylbromid in 30 ml Dioxan gelöst bei 0°C unter Stickstoffatmosphere gegeben. Nach 14 h Rühren bei Raumtemperatur wird mit 400 ml Toluol versetzt, dreimal mit je 200 ml Wasser gewaschen, getrocknet und zum Sirup eingeengt. Das einheitliche Produkt (43.6 g; $CHCl_3$/Essigester 7:1)wird in einer Mischung aus 650 ml Dichlormethan und 144 ml Eisessig gelöst und bei RT mit 8.25 g (0.0225 Mol) Titantetrabromid versetzt. Nach 30 min. werden weitere 1.5 g Titantetrabromid zugegeben. Die Abspaltung der Trityl-Schutzgruppe ist nach 2 h vollständig (DC; $CHCl_3$/Hexan 3:1). Das Reaktionsgemisch wird einmal mit Eiswasser gewaschen und die wäßrige Phase mit Chloroform rückextrahiert.

Die vereinigten org. Phasen werden über Magnesiumsulfat getrocknet, i. vac. eingeengt und mit Toluol destilliert. Aus dem resultierenden Sirup wird zuerst das Tritylbromid mit Hexan kristallisiert. Die Reinigung des Produktes erfolgt säulenchromatographisch (300 g Kieselgel; Petrolether/Essigester 5:1).

Ausbeute 17.24 g (75%)

$[\alpha]_D^{20}$ = + 33.4° (c = 1.25 in $CHCl_3$)

IR ($-N_3$; 2100 $cm^{-1}$)

$^{13}$C-NMR (270 MHz)

C-1 $\delta$ = 62.0 C-2 78.3 . C-3 51.3 ppm

$C_{10}H_{13}N_3O_2$ (207.6) Ber. C 58.3 H 7.0 N 21.2

Gef. C 58.1 H 6.8 N 20.8

Beispiel 4

3-Amino-2-0-benzyl-3-desoxy-sn-glycerin (4):

10 g (48.1 mMol) 3 werden in einer Mischung aus 200 ml Essigester, 100 ml Methanol und 1 ml konz. Ammoniak gelöst. Nach Zugabe von 10g Pd/C (10%) wird 3 h hydriert. Das auf dem Dünnschichtchromatogramm UV-aktive und mit Ninhydrin rotgefärbte Produkt (DC; $CHCl_3$/Methanol/Ammoniak 65:35:3) wird quantitativ erhalten. Es wird abfiltriert, mit Toluol/Methanol 10:1 destilliert. Von dem erhaltenen Sirup (8.7 g) wird ein Teil in das kristalline Hydrochlorid (Methanol/Essigester) überführt und charakterisiert.

Ausbeute 8.3 g (95.2%) Schmp. = 185°C

$[\alpha]_D^{20} = -37.6°$ (c= 1 in $CH_3OH$)

$^{13}$C-NMR (270 MHz, DMSO-$d_6$)

C-1 $\delta$ = 59.9 C-2 75.8 C-3 9.7 ppm

$C_{10}H_{16}Cl\,N\,O_2$ (217,7)

| | | | | |
|---|---|---|---|---|
| Ber. | C 55.17 | H 7,41 | N 6.43 | Cl: 16.29 |
| Gef. | C 55.2 | H 7,39 | N 6.29 | Cl: 16.02 |

Beispiel 5

3-Amino-2-0-benzyl-3-desoxy-glycerin (4): Dargestellt aus 2-0-Benzylglycerin

10 g (54.9) mMol)2-0-Benzylglycerin werden in 80 ml trock. Pyridin gelöst. Nach der Zugabe von 10.47 g (54.9 mMol) p-Toluol-sulfonsäurechlorid wird noch 2 h unter Feuchtigkeitsausschluß gerührt. Die Suspension wird abfiltriert, eingeengt, in Chloroform aufgenommen und zweimal mit Eiswasser gewaschen. Die eingeengte org. Phase wird noch mit Toluol destilliert. Der entstandene Sirup wird in 100 ml DMF gelöst und mit 10 ml konz. Ammoniak-Lösung versetzt. Bei 125°C wird der Lösung innerhalb von 2 h 200 ml konz. Ammoniak-Lösung zugetropft. Nach Einengen wird noch mit Toluol destilliert. Das entstandene Amino-Produkt wird mit verd. Salzsäure in das Hydrochlorid-Derivat überführt, das aus Methanol/Essigester kristallisiert.

Ausbeute 8,6 g (72%)

$^{13}$C-NMR, IR

| | | | | | |
|---|---|---|---|---|---|
| $C_{10}H_{16}Cl\ N\ O_2$ (217.7) | Ber. | C 55.17 | H 7.41 | N 6.43 | Cl 16.29 |
| | Gef. | C 55.08 | H 7.40 | N 6.21 | Cl 16.10 |

Beispiel 6

2-(Bis-(2-chloroethyl)-amino)-5-benzyloxy-tetrahydro-2H-1,3,2-oxazaphosphorin-2-oxid (5a) u. (5b):

31 g (171 mMol) 4 werden in 900 ml trock. Dioxan gelöst. Nach der Zugabe von 59 ml (430 mMol) trock. Triethylamin wird 1/2 h bei Raumtemperatur unter Stickstoffatmosphäre gerührt. Der Lösung werden 52 g (202 mMol) Bis(2-chlorethyl-phosphoramid-dichlorid, gelöst in 300 ml Dioxan, zugegeben, und es wird noch 2 h weitergerührt. Der Suspension werden 500 ml Toluol zugegeben, und das ausgefallene Triethylammoniumchlorid wird abfiltriert. Die Lösung wird i. vac. eingeengt. Der resultierende Sirup wird in Chloroform gelöst und mit Eiswasser gewaschen. Nach Trocknen mit Magnesiumsulfat und Einengen i.vac. wird das Rohprodukt, das jeweils zu 50% aus den exo- und endo-Benzyloxy-Derivaten besteht säulenchromatographisch (600 g Kieselgel ; Elutionsmittel $CHCl_3$/Aceton 4:1) getrennt.

Ausbeute:

(5a) exo 26.5 g (86.6%) $[\alpha]_D^{20}$ =+5.26° (c = 1 in $CHCl_3$)
($^1$H-NMR, IR)

(5b) endo 27.2 g $[\alpha]_D^{20}$ = -10.82° (c = 1 in $CHCl_3$)

$C_{14}H_{21}Cl_2N_2O_3P$ (366.1) Ber. C 45.79 H 5.76 N 7.63
Cl 19.31

Gef. C 45.83 H 5.73 N 7.57
Cl 19.10

Beispiel 7

2-(Bis-(2-chloroethyl)-amino)-5-hydroxy-tetrahydro-2H-1,3,2-oxazaphosphorin-2-oxid (6a) und (6b):

4.2 g (11.5 mMol) (5a) oder (5b) (exo- bzw. endo-Derivate) werden in 40 ml Methanol/Wasser 3:1 gelöst und in Gegenwart von 4.2 g Paladiumkohle (10%) 2 h bei Raumtemperatur hydriert. Dann wird filtriert, nachgewaschen, mit HCl neutralisiert und i. vac. eingeengt. Der Rückstand wird in Aceton aufgenommen. Die anorganische Bestandteile werden abfiltriert. Das Rohprodukt wird in Aceton bei -18°C kristallisiert.

(6a) exo-Derivat

Ausbeute 2.75 g (87%) $[\alpha]_D^{20} = +0.79°$ (c = 1 in $CHCl_3$)

$^1H$-NMR (270 MHz $CD_3OD$):

4H $\delta$ = 3.17 m, 5H 3.88 m 6a-H 4.13 m 6e-H 4.28 m ppm

$J_{6a,\ 6e} = 11.0$, $J_{6a,\ 5} = 6.5$, $J_{6e,\ 5} = 4.0$, $J_{6e,\ 4e} = 2$ Hz

$C_7H_{15}Cl_2N_2O_3P$ (277.0) Ber. C 30.34 H 5.45 N 10.11 Cl 25.54

Gef. C 30.42 H 5.46 N 10.07 Cl 25.30

(6b) endo-Derivat

Ausbeute 2.81 g (89%) $[\alpha]_D^{20} = -7.39°$ (c = 1 in $CHCl_3$)

$^1H$-NMR (270 MHz DMSO-$d_6$)

4H $\delta$ = 2.95 5H 3.67 6a-H 4.2 6e-H 3.93

$J_{6a,\ 6e} = 11.0$, $J_{6a,\ 5} = 2.5$, $J_{6e,\ 5} = 4.5$, $J_{6e,\ 4e} = 1.5$ Hz

$C_7H_{15}Cl_2N_2O_3P$ (277.0)

Ber. C 30.34 H 5.45 N 10.11 Cl 25.54

Gef. C 30.28 H 5.44 N 10.03 Cl 25.26

0072531

**Beispiel 8**

3-Azido-1-O-benzyl-3-desoxy-glycerin (7):

50 g (0.46 Mol) Benzylalkohol gelöst in 600 ml trock. DMF werden bei Raumtemperatur unter $N_2$-Atmosphäre mit 128.8 g (2.3 Mol) KOH-Pulver versetzt. 85.13 g (0.92 Mol) Epichlorhydrin gelöst in 200 ml trock. DMF wird innerhalb von 1 h zugetropft. Nach 3 h wird die Suspension mit 1 l Äther versetzt, und die anorganischen Bestandteile werden abfiltriert. Nach Einengen wird der erhaltene Sirup in Chloroform aufgenommen und mit Eiswasser gewaschen. Die organische Phase wird i. vac. eingeengt und im Hochvakuum bei 100°C 2 h getrocknet. Der mit Benzylakohol verunreinigte Sirup (60.6 g) wird in einem Gemisch aus 1200 ml Ethanol und 300 ml $H_2O$ gelöst, mit 151.15 g (2.32 Mol) Natriumazid versetzt und 2 h bei 80°C erhitzt. Es wird abfiltriert und i. vac. eingeengt. Der Sirup wird in Chloroform gelöst und die Lösung mit Wasser gewaschen. Nach dem Trocknen über Magnesiumsulfat und Einengen wird der Sirup noch 2 h im Hochvakuum getrocknet. Zur Bestimmung der analytischen Werte wird ein Teil säulenchromatographisch gereinigt ($CHCl_3$/Essigester 20:1).

Ausbeute: 60.1 g (78.7%) IR ($-N_3$, 2120 $cm^{-1}$; OH, 3580 $cm^{-1}$ und 3440 $cm^{-1}$)

$^1$H-NMR (100 MHz, DMSO-$d_6$)

1a-H $\delta$= 3.32 m, 1b-H 3.41 m, 2-H 3.83 m, 3a-H 3.33
3b-H 3.38, Ph $CH_2$ 4.49 s, Ph 7.3 m,
OH 5.25 d ppm

$C_{10}H_{13}N_3O_2$ (207.3)

| | | | |
|---|---|---|---|
| Ber. | C 57.96 | H 6.32 | N 20.28 |
| Gef. | C 57.87 | H 6.30 | N 20.10 |

Beispiel 9

3-Azido-1-0-benzyl-3-desoxy-2-0-hexadecyl-glycerin (8):

20 g (96.5 m Mol) 7 gelöst in 200 ml einer Mischung aus trock. Dioxan/tert. Butanol 1:1, 16.25 g (145 m Mol) Kaliumtertiärbutylat und 34 g (105 m Mol) Methansulfonsäurehexadecylester werden bei Feuchtigkeitsausschluß 2.5 h unter Rückfluß erhitzt. Die abgekühlte Suspension wird mit 0.5 l Äther versetzt, und die org. Phase wird zweimal mit je 300 ml Wasser gewaschen. Es wird über Magnesiumsulfat getrocknet und i. vac. eingeengt. Das Produkt wird noch an 300 g Kieselgel chromatographisch ($CHCL_3$/Essigester 30:1) gereinigt.

Ausbeute: 41.0 g (98%) Sirup

IR ($-N_3$, 2120 $cm^{-1}$)

$^{13}C$-NMR (270 MHz $CDCl_3$)

C-1 $\delta$ = 69.5, C-2 77.9 C-3 52.0

Ph $CH_2$ 73.4 ppm

| | | | |
|---|---|---|---|
| $C_{26}H_{45}N_3O_2$ (431.7) | Ber. C 72.34 | H 10.51 | N 9.7 |
| | Gef. C 72.60 | H 10.4 | N 9.4 |

Beispiel 10

3-Azido-1-O-benzyl-3-desoxy-3-O-decyl-glycerin (9):

Darstellung wie 8 (IR, NMR)

$C_{20}H_{33}N_3O_2$ (347.5) Ber. C 69.13 H 9.57 N 12.09

Gef. C 69.02 H 9.60 N 11.92

Beispiel 11

3-Amino-3-desoxy-2-O-hexadecyl-glycerin-hydrochlorid (10):

20 g (46.3 m Mol) 8 werden in einer Mischung aus 200 ml Essigester, 160 ml Methanol und 3.8 ml konz. HCl gelöst und in Gegenwart von 10 g Pd/C (10%) und 0.2 g Pd-Schwarz 24 h hydriert. Anschließend werden noch 2.5 g Pd/C (10%) und 3.8 ml konz. HCl zugegeben, und es wird weitere 3 h hydriert. Zur Aufarbeitung wird mit 100 ml Methanol verdünnt, filtriert, nachgewaschen und i. vac. eingeengt. Das Produkt wird aus Äther/Aceton kristallisiert.

Ausbeute: 12.8 g (81%)

$^{13}C$-NMR (270 MHz, $CD_3OD/CDCl_3$, 2:1)

C-1 $\delta$ = 59.9 C-2 75.8 C-3 97.7 ppm

$C_{19}H_{42}Cl\,N\,O_2$ (377.5) Ber. C 64.83 H 12.03

Cl 10.07 N 3.98

Gef. C 64.54 H 11.90

Cl 9.8 N 3.91

Beispiel 12

3-Amino-3-desoxy-2-O-decyl-glycerin-hydrochlorid (11):

Darstellung wie 10

$^{13}C$-NMR (270 MHz, $CD_3OD/CDCl_3$, 2:1)

C-1 $\delta$ = 61.54 C-2 75.65 C-3 41.64 ppm

$C_{13}H_{30}Cl\ N\ O_2$ (267.8)

| | | |
|---|---|---|
| Ber. | C 58.30 | H 11.29 |
| | Cl 13.24 | N 5.23 |
| Gef. | C 58.41 | H 11.31 |
| | Cl 13.03 | N 5.13 |

## Beispiel 13

2-(Bis-(2-chlorethyl)-amino)-5-hexadecyloxy-tetrahydro-2H-1,3,2-oxazaphosphorin-2-oxid (12a) und (12b):

9 g (23.8 mMol) 10 werden in 250 ml trock. Dioxan gelöst. Nach der Zugabe von 13.2 ml (95.4 mMol) trock. Triethylamin wird 0.5 h bei Raumtemperatur unter Stickstoffatmosphäre gerührt. Der Lösung werden 7.99 g (30.94 mMol) Bis-(2-chlorethyl)phosphoramid-dichlorid, gelöst in 100 ml Dioxan, zugegeben, und es wird noch 3 h weitergerührt. Der Suspension werden 500 ml Toluol zugegeben, und das ausgefallene Triethylammoniumchlorid wird abfiltriert. Die Lösung wird i. vac. eingeengt. Der resultierende Sirup wird in Chloroform gelöst und mit Eiswasser gewaschen. Nach Trocknen mit Magnesiumsulfat und Einengen i. vac. wird das Rohprodukt, das jeweils zu 50% aus den Exo- und Endo-Alkyloxy-Derivaten besteht, säulenchromatographisch (200 g Kieselgel, Elutionsmittel $CHCl_2$/Aceton 4:1) getrennt.

Ausbeute 12a Exo-Derivat
5.3 g (88.77%)

$^1$H-NMR (270 MHz, $CDCl_3$)
H-6a $\delta$ = 4.27, H-6e 4.23, $ClCH_2$ 3.63,
Alkyl $CH_2$ 1.55 und 1.27, $CH_3$ 0.87 ppm

| | | | | |
|---|---|---|---|---|
| $C_{23}H_{47}Cl_2N_2O_3P$ (501.52) | Ber. | C 54.11 | H 9.28 | Cl 13.89 |
| | | N 5.49 | | |
| | Gef. | C 54.3 | H 9.1 | Cl 14.1 |
| | | N 5.6 | | |

12b Endo-Derivat
4.89 g (82%)

$^1$H-NMR (270 MHz, $CDCl_3$), IR

| | | | | |
|---|---|---|---|---|
| $C_{23}H_{47}Cl_2N_2O_3P$ (501.52) | Ber. | C 54.11 | H 9.28 | Cl 13.89 |
| | | N 5.49 | | |
| | Gef. | C 54.2 | H 9.0 | Cl 14.1 |
| | | N 5.5 | | |

**Beispiel 14**

2-(Bis-(2-chlorethyl)-amino)-5-decyloxy-tetrahydro-2H-1,3,2-oxazaphosphorin-2-oxid (13a) und (13b):

Die Darstellung erfolgt wie 12a und 12b.

| Ausbeute: | Exo-Derivat | Endo-Derivat |
|---|---|---|
| | 5.75 g (82%) | 5.75 g (81%) |

$^1H$-NMR (270 MHz, $CDCl_3$), IR

Exo-Derivat:

| | | | | |
|---|---|---|---|---|
| $C_{17}H_{35}Cl_2N_2O_3P$ (417.48) | Ber. | C 48.92 | H 8.45 | Cl 16.99 |
| | | N 6.71 | | |
| | Gef. | C 49.1 | H 8.3 | Cl 17.2 |
| | | N 6.81 | | |

Endo-Derivat:

| | | | | |
|---|---|---|---|---|
| $C_{17}H_{35}Cl_2N_2O_3P$ (417.48) | Ber. | C 48.92 | H 8.45 | Cl 16.99 |
| | | N 6.71 | | |
| | Gef. | C 49.0 | H 8.4 | Cl 17.1 |
| | | N 6.7 | | |

Beispiel 15

5-Benzyloxy-cyclophosphamid(2-(Bis(chloräthyl)-amino)-5-benzyloxy-tetrahydro-2H-1,3,2-oxaza-phosphorin-2-oxid).

A. 1-0-Allyl-2-0-benzyl-rac-glycerin (o,5 Mol) werden in 1,5 l THF gelöst und mit Triäthylamin (o,8 Mol) versetzt. Nach Kühlen auf o° C wird unter einem Stickstoffstrom Methansulfochlorid (o,6 Mol) langsam und unter Rühren eingetropft. Die Vollständigkeit der Reaktion wird dünnschichtchromatographisch nachgewiesen. Man versetzt mit 7oo ml Diisopropyläther und 7oo ml Eiswasser. Die Diisopropyläther-Phase enthält das Produkt und wird über Natriumsulfat getrocknet. Man filtriert und entfernt das Lösungsmittel, wobei ein öliger, leicht gelber Rückstand anfällt. Das Produkt wird ohne weitere Reinigung mit Ammoniak umgesetzt. Anstelle des Racemats können auch die optisch aktiven Isomeren eingesetzt werden.

Der ölige Rückstand wird in 1,2 l DMF aufgenommen und mit 1,6 l 25 %-igem wäßrigem Ammoniak versetzt. Man erhitzt für 24 Stunden auf 6o° C, wonach alles Ausgangsprodukt umgesetzt ist ($R_f$ o,9 für das Ausgangsprodukt; $R_f$ o,4 für das Amin in Chloroform/Methanol/Wasser, 2oo : 15 : 1). Man extrahiert zweimal mit je 7oo ml Chloroform und entfernt das Lösungsmittel am Rotationsverdampfer unter Wasserstrahlvakuum. Das Produkt wird chromatographisch gereinigt. Die Ausbeute an reinem 1-0-Allyl-2-0-benzyl-3-amino-rac-propandiol-(1,2) beträgt 76 %, bezogen auf Allyl-benzyl-glycerin.

Mikroanalyse: $C_{13}H_{19}NO_2$ (221,3o)

| | | | |
|---|---|---|---|
| berechnet: | C 7o,56 | H 8,65 | N 6,33 |
| gefunden: | 7o,41 | 8,55 | 6,13 |

B) 1o g Pd/C-Katalysator (1o % Pd werden in 1oo ml Methanol und o,5 ml konz. $H_2SO_4$ bis zur Sättigung mit Wasserstoff behandelt. Die Wasserstoffaufnahme beträgt etwa 2oo ml. Man leitet unter Rühren für 5 min. einen Stickstoffstrom durch das Katalysatorgemisch, um überschüssigen Wasserstoff zu entfernen. Eine Lösung von nach A.erhaltenem 1-O-Allyl-2-O-benzyl-3-amino-rac-propandiol-(1,2) (o,2 Mol) in 4oo ml Methanol wird mit 4o ml dest. Wasser versetzt, mit konz. $H_2SO_4$ auf einen pH-Wert von etwa 2 eingestellt und zum Katalysator gegeben. Man kocht für 2 Stunden unter Rückfluß, filtriert und versetzt mit 5M Natronlauge bis zur alkalischen Reaktion (pH 1o). 2-O-Benzyl-3-amino-rac-glycerin wird mit Chloroform extrahiert, und die Chloroformphase im Wasserstrahlvakuum vom Lösungsmittel befreit. Der Rückstand wird chromatographisch gereinigt (Rf o,4 für das Ausgangsprodukt; Rf o,1 für 2-O-Benzyl-3-amino-rac-propandiol-(1,2) in Chloroform/Methanol/25 %-igem Ammoniak 2oo:15:1(V/V). Die Ausbeute an 2-O-Benzyl-3-amino-rac-propandiol-(1,2) beträgt 9o %.

<u>Mikroanalyse:</u> $C_{10}H_{15}NO_2$ (181,24)

| | | | | | | |
|---|---|---|---|---|---|---|
| ber.: | C | 66,27 | H | 8,34 | N | 7,73 |
| gef.: | | 66,89 | | 8,19 | | 7,51 |

C) Bis-chloräthylamin (o,1 Mol) werden in 5o ml THF gelöst und tropfenweise zu einer Lösung von $POCl_3$ (o,12 Mol) in 5o ml THF gegeben, die bei o° C mit Triäthylamin (o,12 Mol) versetzt wurde. Man läßt innerhalb einer Stunde auf 2o° C kommen, filtriert vom ausgefallenen Triäthylaminhydrochlorid ab und entfernt das Lösungsmittel nach Zusatz von 5o ml Toluol bei 4o°C im Wasserstrahlvakuum. Der ölige Rückstand wird in 1oo ml THF aufgenommen und bei 25° C tropfenweise mit 3-Amino-2-benzyloxy-propan-1-ol (0,1 Mol) und

Triäthylamin (o,3 Mol) in 1oo ml THF versetzt. Die Vollständigkeit der Reaktion wird dünnschichtchromatographisch verfolgt.

Es entstehen zwei Produkte mit $R_f$-Werten von o,5 und o,6 /Cyclophosphamid o,5 - Fließmittelsystem $CHCl_3/CH_3OH/H_2O$, 2oo/15/1 (v/v)/. Beide Substanzen konnten durch Chromatographie an Kieselgel gereinigt werden. Sie erwiesen sich in der Mikroanalyse identisch und sind Konformationsisomere, wie aus entsprechenden Untersuchungen an Dioxaphosphorinanen (Baran et al., J. Org. Chem. 42 (1977), 226o-2264) hervorgeht. Die Ausbeute an 5-Benzyloxy-cyclophosphamid beträgt 85 %, bezogen auf 3-Amino-2-benzyloxy-propan-1-ol.

Mikroanalyse: $C_{14}H_{21}Cl_2H_2O_3P$ (367,23)

Produkt ($R_f$ o,5)

| | C | H | Cl | N | P |
|---|---|---|---|---|---|
| berechnet: | 45,79 | 5,76 | 19,31 | 7,63 | 8,4 |
| gefunden: | 45,11 | 5,66 | 18,76 | 7,41 | 8,o |

Produkt ($R_f$ o,6)

| | C | H | Cl | N | P |
|---|---|---|---|---|---|
| gefunden: | 45,36 | 5,69 | 18,94 | 7,39 | 8,4 |

Beispiel 16

5-Hydroxy-cyclophosphamid (2-Bis(2-chloräthyl)-amino)-5-hydroxytetrahydro-2H-1,3,2-oxazaphosphorin-2-oxid).

5-Benzyloxy-cyclophosphamid (o,o5 Mol), hergestellt nach Beispiel 15, werden in einem Gemisch aus 2oo ml Methanol und 2oo ml Diisopropyläther gelöst und mit 3 g Pd/C (1o %) versetzt. Unter Wasserstoffatmosphäre wird bei leichtem Überdruck so lange hydriert, bis die Wasserstoffaufnahme beendet ist (Verbrauch ca. 1,2 l Wasserstoff). Man filtriert und entfernt die Lösungsmittel am Rotationsverdampfer. Der ölige Rückstand erstarrt nach längerem Stehen bei o$^{o}$ C. Dünnschichtchromatographisch zeigen sich wie bei 5-Benzyloxy-cyclophosphamid zwei Produkte, die wieder als Konformationsisomere identifiziert werden konnten ($R_f$ o,4 und o,3 in $CHCl_3/CH_3OH/H_2O$, 2oo/15/1 (v/v)). Die Ausbeute an 5-Hydroxy-cyclophosphamid beträgt 95 %.

Mikroanalyse: $C_7H_{15}Cl_2N_2O_3P$ (277,1o)

Produkt ($R_f$ o,4)

| | C | H | Cl | N | P |
|---|---|---|---|---|---|
| berechnet: | 3o,34 | 5,46 | 25,59 | 1o,11 | 11,1 |
| gefunden: | 29,97 | 5,33 | 25,46 | 1o,o1 | 1o,8 |

Produkt ($R_f$ o,3)

| | C | H | Cl | N | P |
|---|---|---|---|---|---|
| gefunden: | 3o,41 | 5,25 | 25,32 | 1o,o7 | 1o,7 |

0072531

Beispiele 17 bis 22

Ausgehend von 5-Hydroxy-cyclophosphamid (Beispiel 15) wurden durch Verätherung auf an sich bekannte Weise hergestellt:

5-Methoxy-cyclophosphamid: $C_8H_{17}Cl_2N_2O_3P$ (MG 291,1)
Nachweis durch Dünnschichtchromatographie

5-Allyloxy-cyclophosphamid: $C_{10}H_{19}Cl_2N_2O_3P$; (MG 317,2)
Nachweis durch Dünnschichtchromatographie

5-Decyloxy-cyclophosphamid: $C_{17}H_{35}Cl_2N_2O_3P$; (MG 417,4)
Nachweis durch Dünnschichtchromatographie

5-Eicosoxy-cyclophosphamid: $C_{27}H_{55}Cl_2N_2O_3P$; (MG 557,6)
Nachweis durch Dünnschichtchromatographie

5-(3-Hydroxy-propoxy)-cyclophosphamid:
$C_{10}H_{21}Cl_2N_2O_4P$; (MG 335,2)
Nachweis durch Dünnschichtchromatographie

5-(3-Benzoxy-propoxy)-cyclophosphamid:
$C_{17}H_{27}Cl_2N_2O_4P$; (MG 425,3)
Nachweis durch Dünnschichtchromatographie.

Beispiele 23 bis 28

Ausgehend von 5-Hydroxy-cyclophosphamid (Beispiel 15) wurden durch Veresterung in an sich bekannter Weise hergestellt:

5-Acetyloxy-cyclophosphamid: $C_9H_{17}Cl_2N_2O_4P$ (MG 319,1)
Nachweis durch Dünnschicht-chromatographie

5-Succinyloxy-cyclophosphamid:
$C_{11}H_{19}Cl_2N_2O_6P$ (MG 377,1)
Nachweis durch Dünnschicht-chromatographie

5-Caprinoyloxy-cyclophosphamid:
$C_{17}H_{33}Cl_2N_2O_4P$ (MG 431,3)
Nachweis durch Dünnschicht-chromatographie

5-Laurinoyloxy-cyclophosphamid:
$C_{19}H_{37}Cl_2N_2O_4P$ (MG 459,4)
Nachweis durch Dünnschicht-chromatographie

5-Palmitoyloxy-cyclophosphamid:
$C_{23}H_{45}Cl_2N_2O_4P$ (MG 515,5)
Nachweis durch Dünnschicht-chromatographie

5-Oleoyloxy-cyclophosphamid: $C_{25}H_{47}Cl_2N_2O_4P$ (MG 541,5)
Nachweis durch Dünnschichtchromatographie

Patentansprüche:

1. Verbindungen der allgemeinen Formel I

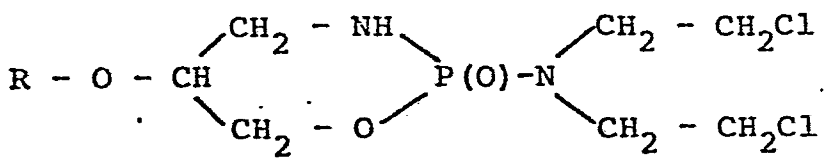

(I)

worin R ein Wasserstoffatom, einen Kohlenwasserstoffrest oder einen heterocyclischen Rest bedeutet.

2. Verbindungen nach Anspruch 1, worin R ein Wasserstoffatom, einen Alkylrest mit 1 bis 26, vorzugsweise 1 bis 16 C-Atomen, der eine oder mehrere Doppel- oder/und Dreifachbindungen oder/und eine oder mehrere OH-Gruppen enthalten kann, oder/und durch Sauerstoffatome unterbrochen sein kann, einen Arylrest oder einen Aralkylrest bedeutet.

3. Verbindungen nach Anspruch 1 oder 2, worin R ein Wasserstoffatom oder eine Hydroxy-alkoxy-gruppe mit 1 bis 6 C-Atomen bedeutet.

4. Verfahren zur Herstellung einer Verbindung der allgemeinen Formel I, d a d u r c h  g e k e n n z e i c h n e t , daß man eine Verbindung der allgemeinen Formel II

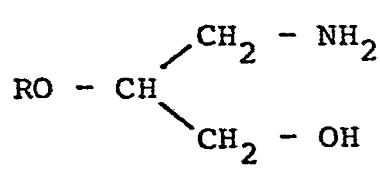

(II)

in der R die in Anspruch 1 angegebene Bedeutung besitzt, in einem organischen Lösungsmittel in Gegenwart einer Base mit N,N-Bis(2-chloräthyl)-phospho-amid-dichlorid oder mit Phosphoroxytrichlorid und Bis(2-chloräthyl)-amin-hydrochlorid umsetzt, und, wenn erwünscht, die erhaltene

Verbindung der allgemeinen Formel I nach an sich bekannten Verfahren in eine Verbindung überführt, in der R einen der anderen in Anspruch 1 angegebenen Rest darstellt.

5. Verfahren nach Anspruch 4, dadurch gekennzeichnet, daß man eine Verbindung der allgemeinen Formel II einsetzt, in der R eine Allyl- oder Benzylgruppe darstellt und, wenn erwünscht, im erhaltenen Produkt die Allyl- bzw. Benzylgruppe abspaltet unter Bildung der Verbindung von Formel I, in der R ein Wasserstoffatom darstellt, und, wenn erwünscht, in dieser Verbindung das Wasserstoffatom nach an sich bekannten Methoden in einen anderen Rest R überführt.

6. Arzneimittel, enthaltend eine Verbindung der allgemeinen Formel I nach Anspruch 1 und übliche, pharmazeutische Konfektionierungs- und/oder Verdünnungsmittel.

Europäisches Patentamt

# EUROPÄISCHER RECHERCHENBERICHT

0072531

Nummer der Anmeldung

EP 82 10 7270.9

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| A | DE - B2 - 2 644 905 (POLSKA AKADEMIA NAUK)<br>* Anspruch *<br>-- | 4 | C 07 F 9/65<br>A 61 K 31/675 |
| A | DE - A1 - 2 317 178 (SHIONOGI)<br>-- | | |
| A | US - A - 3 018 302 (ASTA-WERKE)<br>-- | | |
| A | Chemical Abstracts Band 94, Nr. 25<br>22. Juni 1982<br>Columbus, Ohio, USA<br>SHIONOGI AND CO. LTD. "4-Substituted-2H-1,3,2-oxazaphospholin-2-oxides"<br>Seite 607, Spalte 2, Abstract Nr. 208918d<br>-- | | RECHERCHIERTE SACHGEBIETE (Int. Cl.³)<br>C 07 F 9/65 |
| A | Chemical Abstracts Band 85, Nr. 15<br>11. Oktober 1976<br>Columbus, Ohio, USA<br>P.J. COX et al. "The use of cyclophosphamide analogs in mechanistic studies of the metabolism of cyclophosphamide"<br>Seite 11, Spalte 1, Abstract Nr. 103697s<br>& Adv. Mass Spectrom. Biochem. Med. Nr. 1 1976, Seiten 59 bis 71<br>--<br>./.. | | KATEGORIE DER GENANNTEN DOKUMENTE<br>X: von besonderer Bedeutung allein betrachtet<br>Y: von besonderer Bedeutung in Verbindung mit einer anderen Veröffentlichung derselben Kategorie<br>A: technologischer Hintergrund<br>O: nichtschriftliche Offenbarung<br>P: Zwischenliteratur<br>T: der Erfindung zugrunde liegende Theorien oder Grundsätze<br>E: älteres Patentdokument, das jedoch erst am oder nach dem Anmeldedatum veröffentlicht worden ist<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Berlin | 23-11-1982 | KAPTEYN |

Europäisches Patentamt

# EUROPÄISCHER RECHERCHENBERICHT

EP 82 10 7270.9
- Seite 2 -

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| A | Chemical Abstracts Band 84, Nr. 9<br>1. März 1976<br>Columbus, Ohio, USA<br>P.J. COX et al. "Enzymic basis of the selective action of cyclophosphamide"<br>Seite 10, Spalte 2, Abstract Nr. 53757t<br>& Cancer Res. Band 35, Nr. 12, 1975<br>Seiten 3755 bis 3761<br>---- | | |
| | | | RECHERCHIERTE SACHGEBIETE (Int. Cl.³) |